# EUROPEAN PATENT APPLICATION

(11) **EP 4 751 748 A1**
(43) Date of publication of application: **03.06.2026**
(21) Application number: 23946120.5
(22) Date of filing: 24.07.2023
(51) Int. Cl.: A61M 5/178, A61M 5/24

(54) **FLUID DELIVERY DEVICE AND USE METHOD**

(71) Applicant: Medifabrica Limited, West Hong Kong (HK)
(72) Inventor: RIDLER, Keir, Hong Kong Industrial Building 444-452 Des Voeux Road West Hong Kong (CN); CHAN, Wai Kit Benjamin, Hong Kong Industrial Building 444-452 Des Voeux Road West Hong Kong (CN); FUNG, Ho Wing, Hong Kong Industrial Building 444-452 Des Voeux Road West Hong Kong (CN); WONG, Hau Chi, Hong Kong Industrial Building 444-452 Des Voeux Road West Hong Kong (CN)
(74) Representative: Hoffmann Eitle
(86) International application number: PCT/CN2023/108811
(87) International publication number: WO 2025/020026

(57) **Abstract**

A fluid delivery device (1), comprising: a housing (10), the housing (10) being provided with a mounting opening (101), an accommodating cavity (102) being provided inside the housing (10), and the mounting opening (101) being in communication with the accommodating cavity (102); a cartridge (20), the cartridge (20) being inserted into the mounting opening (101) and detachably mounted within the accommodating cavity (102), and the cartridge (20) being slidable within the accommodating cavity (102) between a first axial position and a second axial position in the axial direction; and a trigger device (30), the trigger device (30) being arranged on the housing (10) and operably connected to the cartridge (20), wherein the trigger device (30) can drive the cartridge (20) to slide from the first axial position to the second axial position, and as the cartridge (20) slides from the first axial position to the second axial position, the cartridge (20) gradually enters the accommodating cavity (102). By means of the fluid delivery device (1) and the use method thereof, different volumes of fluid required for surgery can be accurately delivered and distributed to a target site, preventing accidental delivery of a large amount of fluid to the target site.

## Description

### TECHNICAL FIELD

The present application relates to the field of surgical tools, in particular to a fluid delivery device and a method for using the same.

### BACKGROUND

Fluid delivery devices are often used in surgical operations to deliver a volume of fluid (e.g., medical fluid) to a target position (e.g., a lesion in the human body). Conventional fluid delivery devices are generally disposable devices, which have a predetermined volume and thus can only deliver a predetermined volume of fluid at a time. If the volume of fluid required to be delivered is less than this predetermined volume, fluid may be wasted, increasing the cost of the procedure. Therefore, Conventional disposable fluid delivery devices generally have a small predetermined volume. If the volume of fluid required is more than the predetermined volume, more than one fluid delivery device may be used, leading to unnecessary waste, and increasing cost of the procedure.

Further, with disposable devices, the user cannot precisely control the volume of fluid that is delivered and dispensed to the target position. Although fluid delivery devices including syringes have been proposed to allow the user to control the volume of fluid dispensed, the user is unable to dispense the fluid at a uniform flow rate with such devices including syringes. If the syringe "stick-slips", it may accidentally deliver a large amount of fluid to the target position.

### SUMMARY

Based on this, it is necessary to provide a fluid delivery device and a method for using the same. The fluid delivery device is capable of accurately delivering and dispensing different volumes of fluid required for surgery to a target position and avoiding accidental delivery of large volumes of fluid to the target position.

According to one aspect of the present application, a fluid delivery device is provided. The fluid delivery device includes: a housing provided with a mounting opening, a receiving chamber being disposed in the housing, and the mounting opening being in communication with the receiving chamber; a cartridge inserted into the mounting opening and detachably mounted into the receiving chamber, the cartridge being slidable in the receiving chamber in an axial direction between a first axial position and a second axial position; and a trigger element disposed on the housing and operatively connected to the cartridge. The trigger element is capable of driving the cartridge to slide from the first axial position to the second axial position, and as the cartridge slides from the first axial position to the second axial position, the cartridge gradually enters the receiving chamber.

In the fluid delivery device provided in an embodiment of the present application, the trigger element drives the cartridge to slide from the first axial position to the second axial position, thereby gradually dispensing the fluid at a uniform flow rate. On the one hand, it can accurately deliver and dispense different volumes of fluid, such as a medical fluid, required for surgery to the target position, and on the other hand, it can prevent accidental delivery of a large amount of fluid to the target position. When the cartridge reaches the second axial position, the fluid inside the cartridge is completely dispensed. By replacing a new cartridge, the continuous dispensing of the fluid is achieved.

In an embodiment, the fluid delivery device further includes an end cap connected to the mounting opening. The end cap includes: a through hole, the cartridge being inserted into the through hole, being detachably mounted into the receiving chamber, and bring rotatable between a first rotational position and a second rotational position; and a first projection projecting from an inner wall of the through hole. The cartridge includes: a first slot disposed on an outer wall of the cartridge and extending in the axial direction, the first slot being provided with a second projection, and a second slot disposed on the outer wall of the cartridge and extending in parallel with respect to the first slot. The first projection is slidable along the first slot or the second slot, when the cartridge is located in a first rotational position, the first projection is located in the first slot, and when the cartridge is located in a second rotational position, the first projection is located in the second slot.

In an embodiment, when the cartridge is inserted into the through hole in a first rotational position, and the cartridge slides in a direction of entering the receiving chamber until the first projection abuts against the second projection , the cartridge reaches the first axial position, and when the cartridge is located in the first axial position and in the first rotational position, the cartridge is rotated to a second rotational position and the cartridge is operatively connected to the trigger element .

In an embodiment, when the cartridge is located in the second axial position and in the second rotational position, the cartridge is rotated to the first rotational position and the cartridge is disconnected from the trigger element.

In an embodiment, the first projection is provided with a first step surface on an end facing the mounting opening, the second projection is provided with a second step surface on an end facing the second rotational position, and the second step surface is adapted to abut against the first step surface.

In an embodiment, the first projection is provided with a first inclined surface on an end facing the receiving chamber, the second projection is provided with a second inclined surface on an end away from the second rotational position, and the second inclined surface is adapted to cooperate with the first inclined surface to biases the first projection to release the second projection.

In an embodiment, the first projection is provided with an avoidance groove, when the cartridge slides in a direction away from the receiving chamber until the second projection is in contact with the first projection, the second projection biases the first projection towards the avoidance groove, and the first projection avoids and releases the second projection.

In an embodiment, the fluid delivery device further includes a first reset member disposed in the receiving chamber and abutting against an end of the cartridge, the first reset member biasing the cartridge in a direction away from the receiving chamber.

In an embodiment, the fluid delivery device further includes a carriage disposed in the receiving chamber, an end of the carriage abuts against the cartridge, the other end of the carriage abuts against the first reset member, and the first reset member biases the carriage in a direction toward the cartridge.

In an embodiment, the carriage is provided with a third projection, the housing is provided with a through groove, the through groove extends in the axial direction, and the third projection passes through the through groove and slides along the through groove.

In an embodiment, an inner wall of the through hole of the end cap is provided with at least one first stopper, the outer wall of the cartridge is provided with at least one second stopper, the cartridge is capable of being inserted into the through hole when the first stopper and the second stopper are aligned in the axial direction.

In an embodiment, wherein the inner wall of the through hole is provided with a receiving portion for receiving the second stopper.

In an embodiment, the first stopper is a concave part and the second stop is a convex part, when the convex part and the concave part are aligned in the axial direction, the convex part is capable of entering the concave part.

In an embodiment, the cartridge further includes a rack disposed on an outer wall of the cartridge and extending in the axial direction, and a gear set is arranged in the receiving chamber, and the cartridge is operatively connected to the trigger element through an engagement of the rack with the gear set.

According to another aspect of the present application, a method for using the fluid delivery device according to any of the above-mentioned embodiments is further provided. The method includes:
inserting a cartridge into a through hole of an end cap in a first rotational position, and aligning a first slot with the first through hole;
pushing the cartridge in a direction of entering the through hole until a first projection abuts a second projection, the cartridge reaching a first axial position;
rotating the cartridge to a second rotational position, the cartridge being operatively connected to a trigger element;
operating the trigger element to drive the cartridge to slide toward a second axial position;
when the cartridge reaching the second axial position, rotating the cartridge to the first rotational position, the cartridge being disconnected from the trigger element;
the cartridge being pushed by a first reset element to slide in a direction away from a receiving chamber to the first axial position, the first projection contacting the second projection; and
biasing the second inclined surface against the first inclined surface, causing the first projection to release the second projection, the first projection continuing to slide along the first slot until the cartridge disengages from the receiving chamber.

In an embodiment, before inserting a cartridge into a through hole of an end cap in a first rotational position, and aligning a first slot with the first through hole, the method further includes: aligning a first stopper with a second stopper axially.

### BRIEF DESCRIPTION OF THE DRAWINGS

The above and other purposes, features, and advantages of the present application will become clearer through a more specific description of the preferred embodiments shown in the attached drawings. The same reference numerals in all the drawings indicate the same parts, and the drawings are not intentionally scaled to the actual size, with the focus on demonstrating the main idea of the present application.

By reading the detailed description of non-limiting embodiments with reference to the following drawings, the other features, purposes, and advantages of this application will become more apparent.
FIG. 1 is a schematic partially exploded view of a fluid delivery device according to an embodiment of the present application, with parts of housing omitted;
FIG. 2 is a schematic perspective view of the fluid delivery device shown in FIG. 1;
FIG. 3 is a schematic perspective view of a cartridge according to an embodiment of the present application;
FIG. 4 is another schematic perspective view of the cartridge shown in FIG. 3;
FIG. 5 is a schematic side view of a fluid delivery device according to an embodiment of the present application, with parts of housing omitted and illustrating the cartridges in a first axial position and a second axial position, respectively;
FIG. 6 is a schematic perspective view of an end cap according to an embodiment of the present application;
FIG. 7 is a schematic rear view of a fluid delivery device according to an embodiment of the present application, showing cartridges in a first rotational position and a second rotational position, respectively;
FIG. 8 is a schematic cross-sectional view of a cartridge and an end cap according to an embodiment of the present application, with parts of the end cap omitted;
FIG. 9 is a partially enlarged view of part A in FIG. 8;
FIG. 10 is a schematic perspective view of a carriage according to an embodiment of the present application;
FIG. 11 is a schematic perspective view of a fluid delivery device according to an embodiment of the present application, with the cartridge and other components of the fluid delivery device omitted;
FIG. 12 is a schematic structural view of a cartridge and an end cap according to an embodiment of the present application;
FIG. 13 is a schematic structural view of a cartridge and an end cap according to an embodiment of the present application;
FIG. 14 is a schematic structural view of a cartridge and an end cap according to an embodiment of the present application;
FIG. 15 is a schematic structural view of a cartridge, a trigger element, and a gear set according to an embodiment of the present application;
FIG. 16 is a schematic structural view of a cartridge, a trigger element, and a gear set according to an embodiment of the present application; and
FIG. 17 is a schematic structural view of a cartridge, a trigger element, and a gear set according to an embodiment of the present application.

### DETAILED DESCRIPTION OF THE EMBODIMENTS

In order to facilitate the understanding of the present application, a more comprehensive description of the present application is provided below with reference to the relevant attached drawings. The preferred embodiment of the present application is shown in the attached drawings. However, the present application can be implemented in many different forms, not limited to the embodiments described herein. On the contrary, the purpose of providing these embodiments is to make the disclosure of the present application more thorough and comprehensive.

It should be noted that when a component is considered to be "connected" to another component, it may be directly connected to and integrated with another component, or it may be connected to another component by an intermediate element. The terms "mounting", "an end", "other end", and similar expressions used in the present application are for illustrative purposes only.

Unless otherwise defined, all technical and scientific terms used in the present application have the same meanings as those commonly understood by those skilled in the art. The terms used in the description of the present application are only for the purpose of describing specific embodiments and are not intended to limit the present application. The term "and/or" used in the present application is intended to include any of the listed items and all combinations of them.

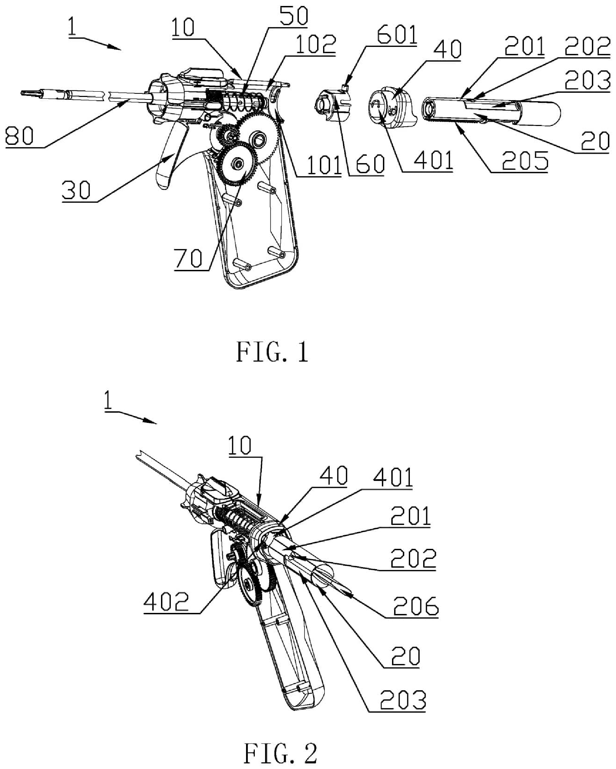

Referring to FIGS. 1 and 2, in an embodiment of the present application, a fluid delivery device 1 is provided. The fluid delivery device 1 includes: a housing 10, a cartridge 20, a trigger element 30, and a delivery tube 80. The housing 10 is provided with a mounting opening 101. A receiving chamber 102 is disposed in the housing 10, and the mounting opening 101 is in communication with the receiving chamber 102. In FIGS. 1 and 2, parts of the housing 10 are omitted for illustrating internal components of the housing 10. The cartridge 20 is inserted into the mounting opening 101 and detachably mounted into the receiving chamber 102. The cartridge 20 is slidable in the receiving chamber 102 in an axial direction between a first axial position and a second axial position. A predetermined volume of the fluid (such as medical fluid) to be dispensed to the target position (such as the patient's lesion) is contained in cartridge 20. The trigger element 30 is disposed on the housing 10 and operatively connected to the cartridge 20. A user is capable of driving the cartridge 20 to slide from the first axial position to the second axial position by operating the trigger element 30. As the cartridge 20 slides from the first axial position to the second axial position, the cartridge 20 gradually enters the receiving chamber 102. The delivery tube 80 is used to deliver the fluid in cartridge 20 to the target position. Optionally, the delivery tube 80 can be selected with appropriate length and inner diameter according to actual needs. In this embodiment, after the fluid in cartridge 20 is fully dispensed to the target position, the cartridge 20 can be disassembled from the fluid delivery device 1 and replaced with a new cartridge 20.

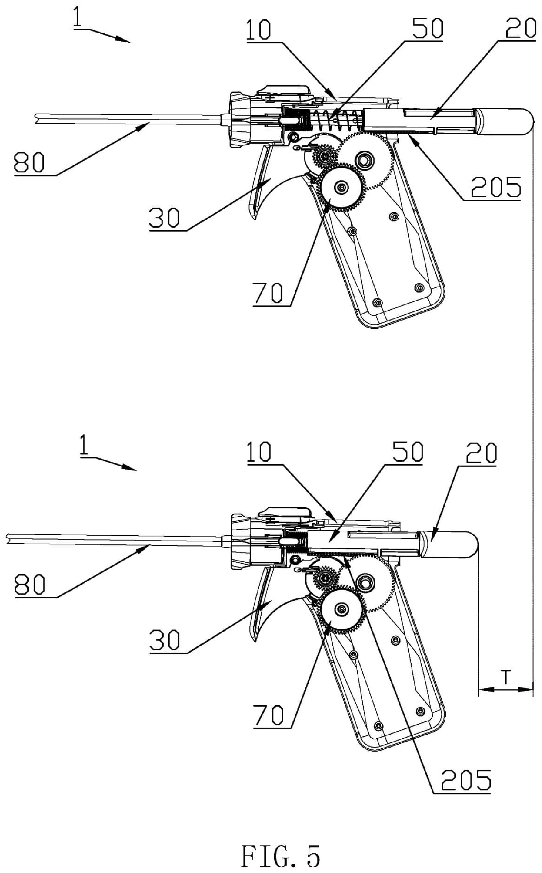

Referring to FIGS. 1 and 5, the upper figure in FIG. 5 shows the cartridge 20 in the first axial position. In this case, the cartridge 20 is inserted into the mounting opening 101, and the fluid delivery device 1 has not yet started dispensing the fluid in the cartridge 20 to the target position. The lower figure in FIG. 5 shows the cartridge 20 in the second axial position. In this case, the liquid in cartridge 20 has been fully dispensed to the target position. The cartridge 20 has a travel T between the first axial position and the second axial position. Those skilled in the art should understand that travel T is related to the volume of cartridge 20. Generally, the cartridge with larger volume has a larger travel T, and vice versa. Optionally, the travel T may be 27mm.

Referring to FIGS. 1 and 2, in an embodiment, the fluid delivery device 1 further includes an end cap 40 connected to the mounting opening 101. The end cap 40 includes: a through hole 401 and a first projection 402. The cartridge 20 is inserted into and extended through the through hole 401, and is detachably mounted into the receiving chamber 102, and the cartridge 20 is rotatable between a first rotational position and a second rotational position. The first projection 402 protrudes from an inner wall of the through hole 401. In this embodiment, the cartridge 20 includes: a first slot 201 and a second slot 203. The first slot 201 is disposed on an outer wall of the cartridge 20 and extends in the axial direction, and the first slot 201 is provided with a second projection 202. The second slot 203 is disposed on the outer wall of the cartridge 20 and extends parallel to the first slot 201. The first projection 402 is slidable along the first slot 201 or the second slot 203. When the cartridge 20 is in the first rotational position, the first projection 402 is located in the first slot 201, and when the cartridge 20 is in the second rotational position, the first projection 402 is located in the second slot 203.

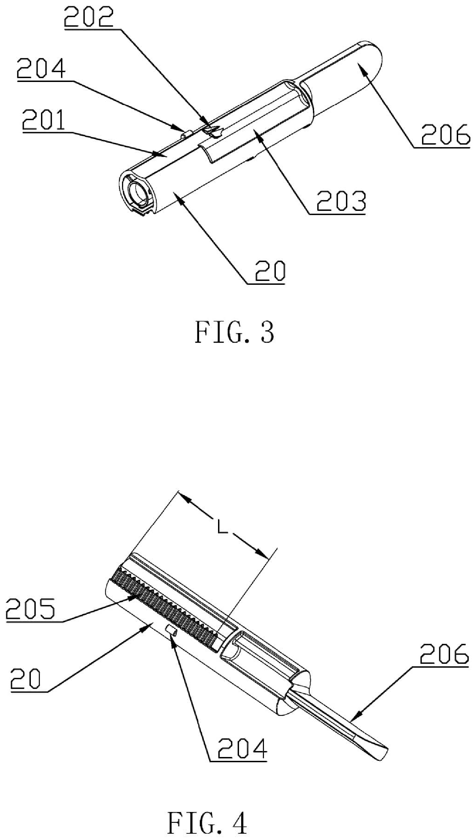

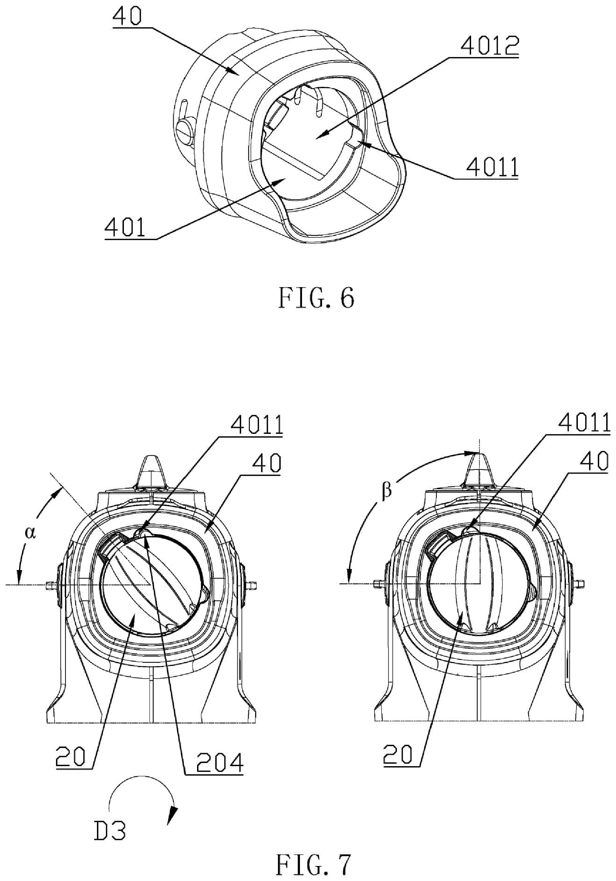

Referring to FIGS. 3 and 4, in an embodiment, the cartridge 20 further includes an operating portion 206. The user inserts the cartridge 20 into the through hole 401 and switches the cartridge 20 between the first rotational position and the second rotational position by operating the operating portion 206. In this embodiment, the operating portion 206 is formed as a sheet shaped. Those skilled in the art should understand that the operating portion 206 may be formed as other suitable shapes according to usage needs, such as rod shaped, ball shaped, etc. The present application does not limit this. Referring to FIGS. 2, 3, and 7 together, the left figure of FIG. 7 shows the cartridge 20 in the first rotational position, and the right figure of Figure 7 shows the cartridge 20 in the second rotational position. In this embodiment, when the cartridge 20 is in the first rotational position, the angle between the operating portion 206 and the horizontal direction is α, and when the cartridge 20 is in the second rotational position, the angle between the operating portion 206 and the horizontal direction is β_{∘} The cartridge 20 rotates from the first rotational position to the second rotational position in the D3 direction. Optionally, α is 45 °. Optionally, β is 90 °. In this embodiment, first, the cartridge 20 is inserted into the through hole 401 at the first rotational position by operating the operating portion 206. Afterwards, the cartridge 20 is pushed towards the second axial position, and when the first projection 402 abuts against the second projection 202, the cartridge 20 reaches the first axial position. In this case, due to the projection 402 abutting against the second projection 202, the sliding of the cartridge 20 towards the second axial position is restricted. By rotating the cartridge 20 through the operating potion 206 (for example, rotating the cartridge 20 in the D3 direction), the cartridge 20 enters the second rotational position, the first projection 402 does not abut against the second projection 202, and the cartridge 20 is operably connected to the trigger element 30. Afterwards, the user can operate the trigger device 30 to gradually slide the cartridge 20 towards the second axial position, in order to gradually dispense the fluid in the cartridge 20 to the target position. When cartridge 20 reaches the second axial position, the fluid in cartridge 20 is fully dispensed to the target position, and cartridge 20 is in the second rotational position. The user rotates the cartridge 20 by the operation portion 206, and the cartridge 20 enters the first rotational position, thereby detaching the cartridge 20 from the operable connection with the trigger element 30. In this case, the cartridge 20 is allowed to be removed from the receiving chamber 102 to replace with a new cartridge 20.

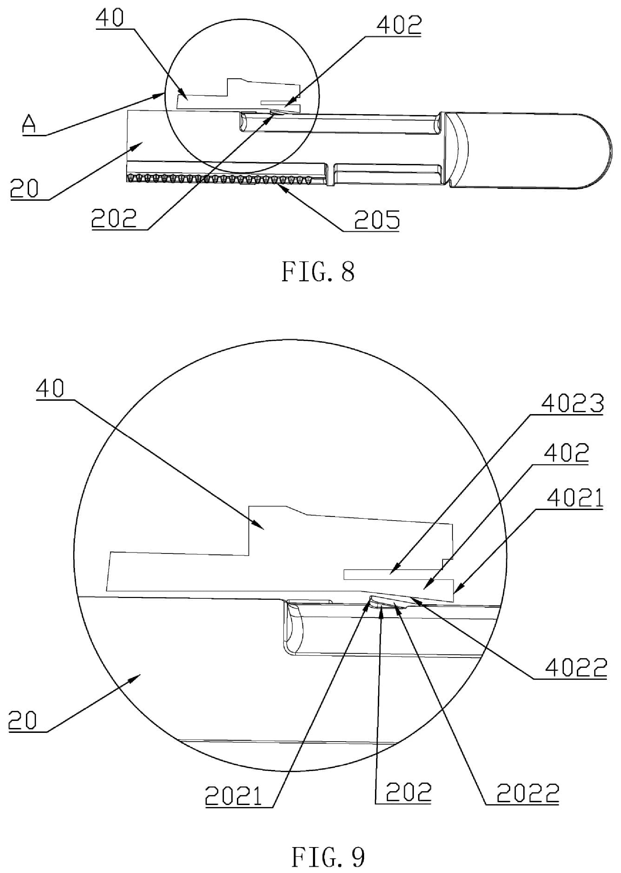

Referring to FIGS. 8 and 9, in an embodiment, an end of the first projection 402 facing the mounting opening 101 (i.e., right end of the first projection 402 in FIG. 9) is defined as an outer end, and an end of the first projection 402 facing the receiving chamber 102 (i.e., left end of the first projection 402 in FIG. 9) is defined as an inner end. The outer end of the first projection 402 is provided with a first step surface 4021, the second projection 202 is provided with a second step surface 2021 on an end facing the second rotational position, and the s second step surface 2021 is adapted to abut against the first step surface 4021. Specifically, in a case that the cartridge 20 is inserted into the through hole 401 and slides towards the second axial position, when the first projection 402 abuts against the second projection 202, the second step surface 2021 abuts against the first step surface 4021, and the cartridge 20 reaches the first axial position. Referring to FIGS. 8 and 9, in an embodiment, the inner end of the first projection 402 is provided with a first inclined surface 4022, the second projection 202 is provided with a second inclined surface 2022 on an end away from the second rotational position, and the second inclined surface 2022 is adapted to cooperate with the first inclined surface 4022 to biases the first projection 402, so as to release the second projection 202. In this embodiment, the first projection 402 is provided with an avoidance groove 4023. Specifically, when the cartridge 20 slides in a direction away from the receiving chamber 102 until the first inclined surface 4022 is in contact with the second inclined surface 2022, the second projection 202 biases the first projection 402 towards the avoidance groove 4023 through the cooperation between the first inclined surface 4022 and the second inclined surface 2022, so that at least a portion of the first projection 402 is deformed (e.g., bent) towards the avoidance groove 4023, the first projection 402 no longer blocks the second projection 202, and the first projection 402 avoids and releases the second projection 202. In this way, the cartridge 20 is allowed to slide towards the direction away from the receiving chamber 102 until it completely disengages from the receiving chamber 102.

Referring to FIG. 5, the fluid delivery device 1 further includes a first reset member 50 disposed in the receiving chamber 102 and abutting against an end of the cartridge 20. The first reset member 50 biases the cartridge 20 in a direction away from the receiving chamber 102. In this embodiment, the first reset member 50 is a spring. Those skilled in the art should understand that the first reset member 50 may also be other suitable elastic devices, such as torsion springs, spring pieces, etc. The present application does not limit this.

Referring to FIGS. 1, 10 and 11, in an embodiment, the fluid delivery device 1 further includes a carriage 60 disposed in the receiving chamber 102, an end of the carriage 60 abuts against the cartridge 20, the other end of the carriage 60 abuts against the first reset member 50 (for example, spring), and the first reset member 50 biases the carriage 60 in a direction toward the cartridge 20. Specifically, when the cartridge 20 is in the second axial position and the second rotational position, when the user rotates the cartridge 20 to the first rotational position, the first reset member 50 pushes the cartridge 20 towards the direction away from the receiving chamber 102 through the carriage 60, thereby eliminating the need for the user to manually slide the cartridge 20 towards the direction away from the receiving chamber 102.

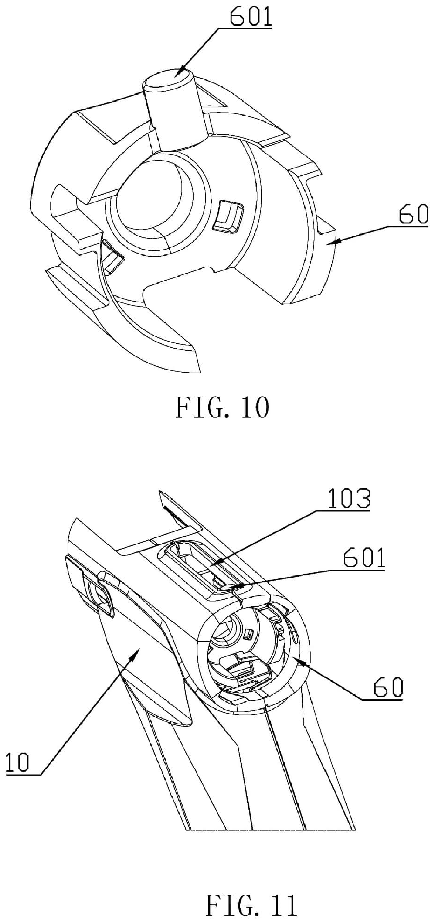

Referring to FIGS. 10 and 11, in an embodiment, the carriage 60 is provided with a third projection 601. The housing 10 is provided with a through groove 103 extending in the axial direction. The third projection 601 extends through and slides along the through groove 103. Furthermore, the third projection 601 extends through the through groove 103, allowing the user to observe an axial position of the third projection 601 to obtain the current axial position of the carriage 60 and evaluate the axial position of the cartridge 20. Optionally, the portion of the third projection 601 exposed outside the through groove 103 is provided with a color indication portion to facilitate the user's observation of the axial position of the projection 601.

Referring to FIGS. 6 and 7, an inner wall of the through hole 401 of the end cap 40 is provided with at least one first stopper 4011, the outer wall of the cartridge 20 is provided with at least one second stopper 204. The cartridge 20 is capable of being inserted into the through hole 401 at the first rotational position, when the first stopper 4011 and the second stopper 204 are aligned in the axial direction. In this embodiment, the first stopper 4011 is a concave part and the second stop 204 is a convex part, when the convex part and the concave part are aligned in the axial direction, the convex part is capable of entering the concave part and engaging in the concave part. Engaging the convex part to the concave part limits the axial rotation of the cartridge 20. The inner wall of the through hole 401 is provided with a receiving portion 4012 for receiving the convex part. Specifically, the concave part has an extension length in the axial direction. When the cartridge 20 is inserted into the through hole 401 in the first rotational position (i.e., the convex part and concave part are aligned axially, and the convex part enters the concave part) and the sliding travel towards the second axial position exceeds the extension length, the convex part detaches from the concave part. In this case, the convex part enters the receiving portion 4012, and the cartridge 20 is allowed to rotate around the axial direction. It should be noted that in this embodiment, although the first stopper 4011 is a concave portion and the second stopper 204 is a convex portion, this is only exemplary. In other embodiments, the first stopper may be the convex portion, and the second stopper 204 may be a concave portion.

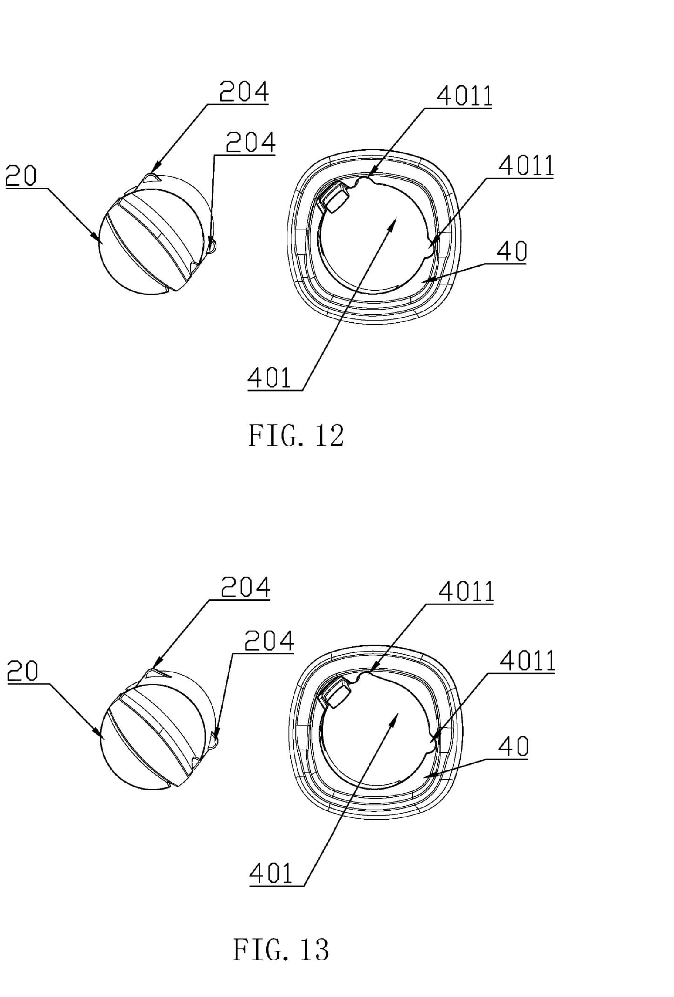

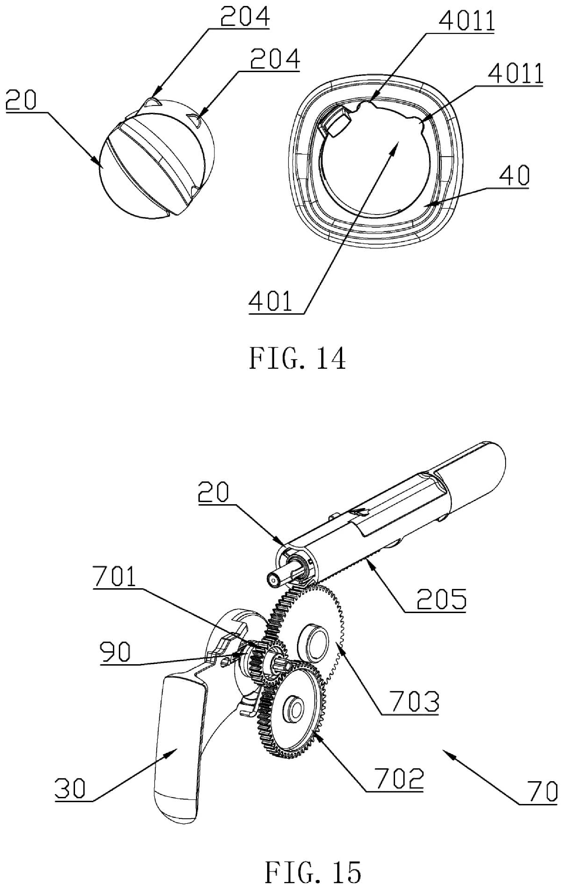

Referring to FIGS. 12 to 14, optional embodiments of the first stopper 4011 and the second stopper 204 are illustrated. Comparing the embodiments shown in FIGS. 12 and 13, in the embodiment shown in FIG. 13, the second stopper 204 has a larger volume. Comparing the embodiments shown in FIGS. 12 and 14, in the embodiment shown in FIG. 14, the circumferential distance between the two second stopper 204 is smaller. By setting the first stopper 4011 and the second stopper 204, a "fool-proofing" function for mounting of cartridge 20 is achieved. Specifically, when the first stopper 4011 and the second stopper 204 are aligned axially, the cartridge 20 is in the first rotational position. Only when the first stopper 4011 and the second stopper 204 are aligned axially can the cartridge 20 be inserted into the through hole 401. It ensures that cartridge 20 can only be inserted into through hole 401 at the first rotational position. Those skilled in the art should understand that the shapes, positions, and quantities of the first stopper 4011 and the second stopper 204 shown in FIGS. 12 to 14 are only exemplary. Those skilled in the art can choose any suitable shapes, positions, or quantities for the first stopper 4011 and the second stopper 204 according to actual needs. For example, in an embodiment, only one first stopper 4011 and one second stopper 204 are provided. For example, in an embodiment, three first stoppers 4011 and three second stoppers 204 are provided. For example, in an embodiment, the first stopper 4011 and the second stopper 204 are formed as semicircles, triangles, or rectangles.

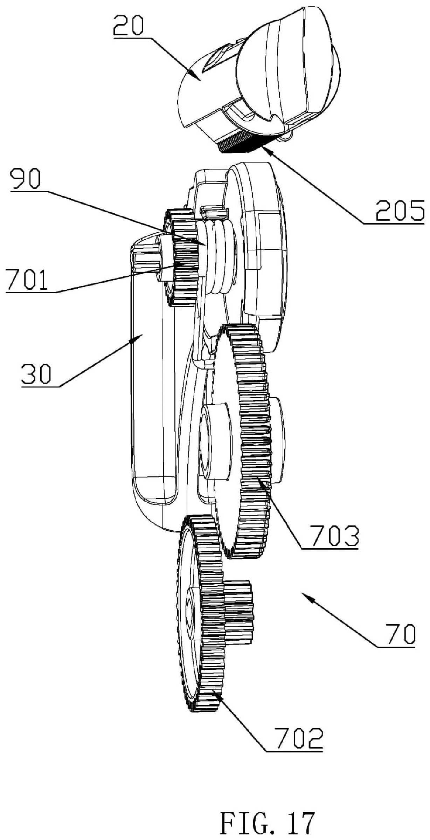

Referring to FIGS. 15 to 17, in an embodiment, the cartridge 20 further includes a rack 205 disposed on an outer wall of the cartridge 20 and extending in the axial direction. A gear set 70 is arranged in the receiving chamber 102, and the cartridge 20 is operatively connected to the trigger element 30 through an engagement of the rack 205 with the gear set 70. Specifically, the trigger element 30 includes a trigger, which is pivotably arranged on the housing 10. The gear set 70 includes a first gear 701, a second gear 702, and a third gear 703. The first gear 701 is fixedly connected to a pivot shaft of the trigger and can pivot with the trigger together. The second gear 702 is pivotably arranged in the receiving chamber 102 and is engaged with the first gear 701. The third gear 703 is pivotably arranged in the receiving chamber 102, and is engaged with the second gear 702 and the rack 205. When the user presses the trigger to pivot it, the first gear 701 pivots along with the trigger. Through the transmission of the second gear 702, third gear 703, and rack 205, the pivot motion of the trigger towards D1 direction is converted into an axial sliding of the cartridge 20 towards D2 direction (i.e., towards the second axial position). In this way, by pressing the trigger, the cartridge 20 is driven to slide towards the second axial position, and the fluid inside the cartridge 20 is gradually dispensed to the target position.

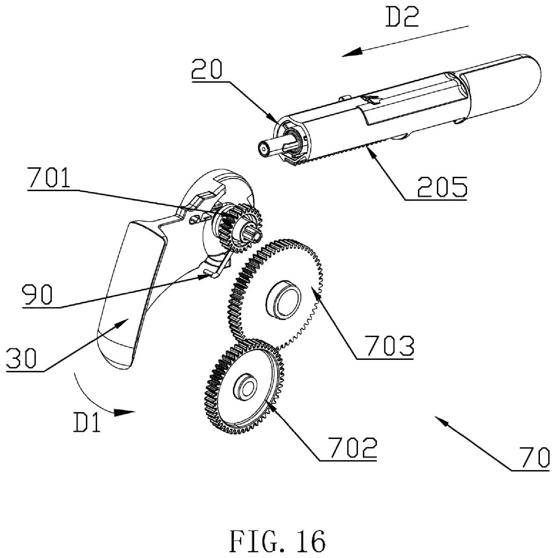

Referring to FIG. 16, in an embodiment, the fluid delivery device 1 further includes a second reset member 90, which is connected to the trigger element 30 and is suitable for biasing the trigger element 30 towards an initial position. Optionally, the second reset member 90 is a torsion spring. After the user removes the pressure on the trigger element 30, under the action of the torsion spring, the trigger element 30 returns to its initial position. Optionally, the trigger element 30 is connected to the first gear 701 through a unidirectional connection mechanism. Only when the trigger element 30 pivots in the D1 direction, the first gear 701 pivots along with the trigger element 30, and when the trigger element 30 pivots in a direction opposite to D1 (for example, when the triggering device 30 returns to its initial position under the action of the torsion spring), the first gear 701 does not pivot along with the trigger element 30. This unidirectional connection mechanism is well-known to those skilled in the art, such as a ratchet mechanism, and this application does not further describe it.

In an embodiment of the present application, a method for using the fluid delivery device 1 according to any above-mentioned embodiment is provided. The method includes:
in Step 101: inserting a cartridge 20 into a through hole 401 of an end cap 40 in a first rotational position, and aligning a first slot 201 with the first through hole 401;
in Step 102: pushing the cartridge 20 in a direction of entering the through hole 401 until a first projection 402 abuts a second projection 202, the cartridge 20 reaching a first axial position;
in Step 103: rotating the cartridge 20 to a second rotational position, the cartridge 20 being operatively connected to a trigger element 30;
in Step 104: operating the trigger element 30 to drive the cartridge 20 to slide toward a second axial position;
in Step 105, when the cartridge 20 reaching the second axial position, rotating the cartridge 20 to the first rotational position, the cartridge 20 being disconnected from the trigger element 30;
in Step 106, the cartridge 20 being pushed by a first reset element 50 to slide in a direction away from a receiving chamber 102 to the first axial position, the first projection 402 contacting the second projection 202; and
in Step 107: biasing the second inclined surface 2022 against the first inclined surface 4022, causing the first projection 402 to release the second projection 202, the first projection 402 continuing to slide along the first slot 201 until the cartridge 20 disengages from the receiving chamber 102.

In an embodiment, before step 101, the method further includes:
in Step 100: aligning a first stopper 4011 with a second stopper 204 axially.

The various technical features of the above embodiments can be combined arbitrarily. To make the description concise, all possible combinations of each technical feature in the above embodiments have not been described. However, as long as there is no contradiction in the combination of these technical features, they should be considered within the scope of this description.

The above embodiments only express several embodiments of the present application, and their descriptions are more specific and detailed, but cannot be understood as limiting the patent scope of the present application. It should be pointed out that for Those skilled in the art, several modifications and improvements can be made without departing from the concept of the present application, all of which fall within the protection scope of present application. Therefore, the protection scope of present application should be based on the attached claims.

## Claims

1. A fluid delivery device (1) comprising:
a housing (10) provided with a mounting opening (101), wherein a receiving chamber (102) is disposed in the housing (10), wherein the mounting opening (101) is in communication with the receiving chamber (102);
a cartridge (20) inserted into the mounting opening (101) and detachably mounted into the receiving chamber (102), wherein the cartridge (20) is slidable in the receiving chamber (102) in an axial direction between a first axial position and a second axial position; and
a trigger element (30) disposed on the housing (10) and operatively connected to the cartridge (20),
wherein the trigger element (30) is capable of driving the cartridge (20) to slide from the first axial position to the second axial position, and as the cartridge (20) slides from the first axial position to the second axial position, the cartridge (20) gradually enters the receiving chamber (102).

2. The fluid delivery device (1) of claim 1, further comprising an end cap (40) connected to the mounting opening (101),
wherein the end cap (40) comprises:
a through hole (401), wherein the cartridge (20) is inserted into the through hole (401), is detachably mounted into the receiving chamber (102), and is rotatable between a first rotational position and a second rotational position; and
a first projection (402) projecting from an inner wall of the through hole (401),
wherein the cartridge (20) comprises:
a first slot (201) disposed on an outer wall of the cartridge (20) and extending in the axial direction, the first slot (201) being provided with a second projection (202), and
a second slot (203) disposed on the outer wall of the cartridge (20), and extending in parallel with respect to the first slot (201),
wherein the first projection (402) is slidable along the first slot (201) or the second slot (203), when the cartridge (20) is located in a first rotational position, the first projection (402) is located in the first slot (201), and when the cartridge (20) is located in a second rotational position, the first projection (402) is located in the second slot (203).

3. The fluid delivery device (1) of claim 2,
wherein when the cartridge (20) is inserted into the through hole (401) in a first rotational position, and the cartridge (20) slides in a direction of entering the receiving chamber (102) until the first projection (402) abuts against the second projection (202), the cartridge (20) reaches the first axial position, and
wherein when the cartridge (20) is located in the first axial position and in the first rotational position, the cartridge (20) is rotated to a second rotational position and the cartridge is operatively connected to the trigger element (30).

4. The fluid delivery device (1) of claim 3,
wherein when the cartridge (20) is located in the second axial position and in the second rotational position, the cartridge (20) is rotated to the first rotational position and the cartridge (20) is disconnected from the trigger element (30).

5. The fluid delivery device (1) of any one of claims 1 to 4,
wherein an outer end of the first projection (402) is provided with a first step surface (4021), the second projection (202) is provided with a second step surface (2021) on an end facing the second rotational position, and the second step surface (2021) is adapted to abut against the first step surface (4021).

6. The fluid delivery device (1) of any one of claims 1 to 4,
wherein an inner end of the first projection (402) is provided with a first inclined surface (4022), the second projection (202) is provided with a second inclined surface (2022) on an end away from the second rotational position, and the second inclined surface (2022) is adapted to cooperate with the first inclined surface (4022) to biases the first projection (402) to release the second projection (202).

7. The fluid delivery device (1) of claim 6,
wherein the first projection (402) is provided with an avoidance groove (4023), when the cartridge (20) slides in a direction away from the receiving chamber (102) until the second projection (202) is in contact with the first projection (402), the second projection (202) biases the first projection (402) towards the avoidance groove (4023), and the first projection (402) avoids and releases the second projection (202).

8. The fluid delivery device (1) of claim 1,
wherein the fluid delivery device (1) further comprises a first reset member (50) disposed in the receiving chamber (102) and abutting against an end of the cartridge (20), the first reset member (50) biasing the cartridge (20) in a direction away from the receiving chamber (102).

9. The fluid delivery device (1) of claim 8,
wherein the fluid delivery device (1) further comprises a carriage (60) disposed in the receiving chamber (102), an end of the carriage (60) abuts against the cartridge (20), the other end of the carriage (60) abuts against the first reset member (50), and the first reset member (50) biases the carriage (60) in a direction toward the cartridge (20).

10. The fluid delivery device (1) of claim 8,
wherein the carriage (60) is provided with a third projection (601), the housing (10) is provided with a through groove (103), the through groove (103) extends in the axial direction, and the third projection (601) passes through the through groove (103) and slides along the through groove (103).

11. The fluid delivery device (1) of claim 2,
wherein an inner wall of the through hole (401) of the end cap (40) is provided with at least one first stopper (4011), the outer wall of the cartridge (20) is provided with at least one second stopper (204), the cartridge (20) is capable of being inserted into the through hole (401) when the first stopper (4011) and the second stopper (204) are aligned in the axial direction.

12. The fluid delivery device (1) of claim 11,
wherein the inner wall of the through hole (401) is provided with a receiving portion (4012) for receiving the second stopper (204).

13. The fluid delivery device (1) of claim 12,
wherein the first stopper (4011) is a concave part and the second stop (204) is a convex part, when the convex part and the concave part are aligned in the axial direction, the convex part is capable of entering the concave part.

14. The fluid delivery device (1) of claim 1,
wherein the cartridge (20) further comprises a rack (205) disposed on an outer wall of the cartridge (20) and extending in the axial direction, and a gear set (70) is arranged in the receiving chamber (102), and the cartridge (20) is operatively connected to the trigger element (30) through an engagement of the rack (205) with the gear set (70).

15. A method for using the fluid delivery device (1) according to any one of claims 1 to 14, comprising:
inserting a cartridge (20) into a through hole (401) of an end cap (40) in a first rotational position, and aligning a first slot (201) with the first through hole (401);
pushing the cartridge (20) in a direction of entering the through hole (401) until a first projection (402) abuts a second projection (202), the cartridge (20) reaching a first axial position;
rotating the cartridge (20) to a second rotational position, the cartridge (20) being operatively connected to a trigger element (30);
operating the trigger element (30) to drive the cartridge (20) to slide toward a second axial position;
when the cartridge (20) reaching the second axial position, rotating the cartridge (20) to the first rotational position, the cartridge (20) being disconnected from the trigger element (30);
the cartridge (20) being pushed by a first reset element (50) to slide in a direction away from a receiving chamber (102) to the first axial position, the first projection (402) contacting the second projection (202); and
biasing the second inclined surface (2022) against the first inclined surface (4022), causing the first projection (402) to release the second projection (202), the first projection (402) continuing to slide along the first slot (201) until the cartridge (20) disengages from the receiving chamber (102).

16. The method of claims 15,
before inserting a cartridge (20) into a through hole (401) of an end cap (40) in a first rotational position, and aligning a first slot (201) with the first through hole (401), further comprising:
aligning a first stopper (4011) with a second stopper (204) axially.
